(19) Europäisches Patentamt / European Patent Office / Office européen des brevets

(11) **EP 4 374 709 A1**

(12) **EUROPEAN PATENT APPLICATION**
published in accordance with Art. 153(4) EPC

(43) Date of publication:
**29.05.2024 Bulletin 2024/22**

(21) Application number: **21950383.6**

(22) Date of filing: **20.07.2021**

(51) International Patent Classification (IPC):
**A23L 33/18** (2016.01)     **A23J 3/34** (2006.01)
**A61K 38/17** (2006.01)     **C12P 21/06** (2006.01)

(52) Cooperative Patent Classification (CPC):
**A23J 3/34; A23L 33/18; A61K 38/17; C12P 21/06**

(86) International application number:
**PCT/CL2021/050064**

(87) International publication number:
**WO 2023/000115 (26.01.2023 Gazette 2023/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Pontificia Universidad Católica de Chile
Santiago (CL)**

(72) Inventors:
• **FELLENBERG PLAZA, María Angélica
Santiago (CL)**
• **PESLERBES, Marie Celine Veronique
Santiago (CL)**
• **IBÁÑEZ ALFARO, Rodrigo Ignacio
Madison, WI 53705, Wisconsin Madison,
Wisconsin (US)**

(74) Representative: **Pons
Glorieta Rubén Darío 4
28010 Madrid (ES)**

(54) **METHOD FOR PRODUCING BIOACTIVE PEPTIDES OBTAINED FROM WHEY USING ENZYMES
OF PLANT ORIGIN**

(57)     The present invention is intended for the food sector, specifically the field of bioactive compounds obtained from food products, and relates to the provision of a new method for producing peptides with anti-hypertensive activity, comprising treating whey from cow's milk with plant proteases. The present invention also relates to a product containing the bioactive peptides and to the uses of said product to formulate foods that allow blood pressure to be maintained at acceptable levels in order to reduce the risk of suffering from diseases related to high blood pressure.

EP 4 374 709 A1

**Description**

**TECHNICAL FIELD**

This invention is framed within the food sector, specifically in the field of bioactive compounds obtained from food products. It provides a new process for producing antihypertensive-activity peptides, aimed to treating cow's milk whey with vegetable proteases. Furthermore, this invention deals with a product containing said bioactive peptides and with uses of said product for manufacturing food aimed to maintaining blood pressure at acceptable levels, in order to reduce the risk of high blood pressure diseases.

**BACKGROUND & PRIOR ART**

Functional foods are defined as products providing benefits beyond basic nutrition. These are a variety of components aimed to reduce diseases and promote health. Functional ingredients add specific features to food, such as antioxidant, antiinflammatory, antihypertensive, antimicrobial, anticancer properties, among others.

One of the main industry incentives that allows appearance of a series of functional ingredients in recent times is to take advantage of by-products from different production processes. Functional ingredients with different properties obtained from waste coming from fruit and vegetable, grain processing, brewing and wine, meat and dairy industries have been described. (Prathamesh Bharat Helkar, AK Sahoo and NJ Patil. 2016. Review: Food Industry By-Products used as a Functional Food Ingredients. Int J Waste Resour 2016, 6:3, DOI: 10.4172/2252-5211.1000248).

Whey is a by product from cheese industry. It is obtained from enzymatic coagulation of caseins (CN), containing approximately 50% of milk nutrients. It is deemed as a potential source of high value-added products and ingredients. Nearly 50% of whey is estimated to be eliminated during the production process, thus generating a potential environmental contamination, due to its high content of organic matter. This scenario can generate eutrophication problems in water bodies where such product is discharged. Therefore, currently the industry must treat this waste or turn them into value-added processed products.

In the market there are whey-derived products, such as powder products. These are directly produced from whey that can be purchased by the animal industry, food industry and final consumers to be used as nutritional supplements. Other whey-derived products are those obtained by means of biotechnology. Whey is used as raw material in order to turn active whey compounds into other added value molecules. Whey has a high content of various substances, with a great diversity of proteins, such as $\beta$-lactoglobulin, $\alpha$-lactalbumin, immunoglobulin, bovine serum albumin, lactoferrin and lactoperoxidase. (Prathamesh Bharat Helkar, AK Sahoo y NJ Patil. 2016. Review: Food Industry By-Products used as a Functional Food Ingredients. Int J Waste Resour 2016, 6:3, DOI: 10.4172/2252-5211.1000248), which may be used as raw material to produce bioactive compounds derived from proteins, or bioactive peptides.

Production of bioactive peptides consists of controlled enzymatic hydrolysis of the proteins contained in the serum by using protease enzyme(s). Among proteases available in the state of the art are those of animal origin, such as pepsin, trypsin and chymotrypsin that have been described as the most used enzymes in several studies. (Korhonen & Pihlanto. 2006. Food-derived Bioactive Peptides - Opportunities for Designing Future Foods. Current Pharmaceutical Design 9(16):1297-308). Although these enzymes have been used to produce bioactive peptides, there is a limited amount of bioactive peptides that can be produced from whey by using conventional enzymes, such as pepsin, trypsin and chymotrypsin. (Tavares & Malcata. 2013, Whey Proteins as Source of Bioactive Peptides Against Hypertension. DOI: 10.5772/52680).

Currently there is a need, in this technical field, to develop new enzymatic hydrolysis processes involving use of microbial/vegetable proteases, in order to find novel bioactive peptides. Both types of enzymes have a number of characteristics that are different from animal digestive proteases, thus offering other optimal ranges of temperature, pH and stability. Additionally, vegetable enzymes have been described to have different preferences for substrates. These traits make them more interesting to be tested for creating bioactive peptides.

A number of biological activities produced by bioactive peptides obtained from whey hydrolysates have been described. Among the biological activities that these bioactive peptides have been described to possess are antimicrobial, anticancer and also antihypertensive properties.

Cardiovascular diseases (heart attacks and strokes among others) are the leading cause of death in the world. Hypertension is the first risk factor (WHO, 2018). Hypertension, also known as high blood pressure, is defined as the condition in which blood pushes against arterial walls, and it is high enough to cause long-term health problems. It causes damages on arteries and other organs, such as the heart, kidneys, retina and brain, among others. Its prevalence has increased in recent decades, especially in developed countries, mainly due to an inadequate lifestyle, including sedentary lifestyle, smoking, alcohol or inadequate diets (WHO, 2018). Although chemical treatments exist, the state of the art teaches that treatments in this technical field focus on developing alternative treatments, based on natural ingredients.

Blood pressure is controlled and regulated by angiotensin-I converting enzyme (ACE). This enzyme participates in the renin-angiotensin system. ACE exerts activity on blood pressure by activating Angiotensin I into Angiotensin II. Angiotensin II acts as a vasoconstrictor and increases blood pressure. ACE also plays another role in the renin-angiotensin system by deactivating bradykinin. Without ACE, bradykinin acts as a vasodilator and helps to lower blood pressure. With ACE, bradykinin is deactivated: inactive fragments are created and bradykinin does not perfom its regulatory role. ACE inhibitors and angiotensin receptor blockers are generally used to control hypertension. A combination of drugs is often used aimed to optimize treatment and also to minimize side effects, such as fatigue, nausea, dizziness or congenital malformations in case of pregnancy. (Torruco-Uco et al., 2009; Angiotensin-I converting enzyme inhibitory and antioxidant activities of protein hydrolysates from Phaseolus lunatus and Phaseolus vulgaris sedes. Food Science and Technology. Volume 42 (10): 1597-1604). Treatment complexity and associated risks made it necessary to find alternatives. One of these is using functional foods aimed to regulate antihypertensive activity.

Over the years, some studies have proved that serum-derived peptides exert an ACE-inhibitory activity. By inhibiting ACE activity, angiotensin I is not turned into angiotensin II and bradykinin remains biologically active, thus regulating hypertension. ACE-inhibitory activity is estimated by using the $IC_{50}$, defined as the minimum concentration of the peptide necessary to inhibit 50% of ACE activity. (Ben Henda et al., 2013, Measuring Angiotensin-I Converting Enzyme Inhibitory Activity by Micro Plate Assays: Comparison Using Marine Cryptides and Tentative Threshold Determinations with Captopril and Losartan. J. Agric. Food Chem. 2013, 61, 45, 10685-10690). The first antihypertensive-activity peptide was isolated from Brazilian cobra venom in 1965. In time, several raw materials were investigated as possible sources for producing antihypertensive peptides, such as fish, spinach, wine or milk. (Torruco-Uco et al., 2009; Angiotensin-I converting enzyme inhibitory and antioxidant activities of protein hydrolysates from Phaseolus lunatus and Phaseolus vulgaris sedes. Food Science and Technology. Volume 42 (10): 1597-1604). As for whey, the most potent inhibitor known and described in the literature is the f-peptide (142-148) derived from β-lactoglobulin, obtained from digestive enzymes (pepsin, trypsin, and chymotrypsin). Several other ACE-inhibitory peptides have been reported; however, the exact molecular mechanisms by which peptides exert their antihypertensive activity are not well understood yet. Furthermore, the processes by which such antihypertensive-activity peptides have been obtained have mainly used animal digestive proteases, such as pepsin and trypsin. There is not much precedent for the use of vegetable proteases, such as bromelain and ficin for producing antihypertensive-activity peptides.

In order to assess the merits of the invention described herein, a brief summary of the most relevant documents existing in the art is presented.

CN111944009A describes an antihypertensive composition derived from Salicornia bigelovii. This document first describes a method for obtaining a protein composition derived from this plant. Then a process is described wherein said protein composition is treated with bromelain in order to produce an antihypertensive-activity composition. The reaction conditions for bromelain were as follows: (1) 20 mM PBS buffer; (2) reaction pH of 6.5; (3) enzyme concentration 10 mg/mL; (4) reaction time was 12 hours.

JP2020097535A describes new compositions for reducing blood pressure. The composition is stated to be an enzymatic degradation product as the active ingredient, which is preferably an enzymatic degradation product, obtained from whey. The active ingredient is described as the GTWY-sequence peptide. The proteolytic enzyme used to produce that bioactive peptide is not specifically described, but general reaction conditions are indicated: (1) temperature between 30°C and 70°C; (2) reaction time between 1 and 12 hours; (3) reaction stopping step at a temperature between 80°C and 90°C for 5 to 30 min; (4) ultrafiltration by using membranes; (5) lyophilization of the product; and (6) the pH of the reaction is described to be between 4 and 9.

CN108178785A describes peptides obtained from sheep milk serum, which have an antihypertensive activity. A production process is described with the following steps: (1) adding water to sheep whey protein powder; (2) treating said blend with ultrasonic waves; (3) adding proteases to the blend in order to perform enzymatic hydrolysis; (4) heating the blend at a certain temperature (between 50°C and 60°C) during around 0.5 and 2 hours; (5) stopping the reaction by heating said reaction at 90°C - 100°C for 10 to 20 min; (6) cool, filter and separate the liquid; (7) two-step ultrafiltration aimed to purify the peptides, using 5 kDa membranes; and (8) lyophilize the sample.

KR20110089705A describes a whey peptide hydrolysate having antihypertensive activity. The production process of said hydrolysate comprises the following steps: (1) mix whey protein powder with commercial enzyme protamex (Novozymes); (2) react this blend at a pH of 6.5, at 45°C for 6 hours; (3) heat the blend aimed to stop the reaction, at 100°C for 15 mins; (4) centrifuge for 20 mins, at 3,000 RPMs; (5) lyophilize the samples.

JP2002238462A describes whey protein hydrolysates that have good aroma, high thermal stability in the acidic region, reduced buffering capacity and do not inhibit gelation.

GB966857A describes a pasteurized whey hydrolysate obtained by means of enzymatic hydrolysis. The production method involves use of pancreatic enzymes, trypsin, pepsin, ficin, papain and proteins derived from *Aspergillus oryzae.*

Mazorra-Manzano, M., et al. 2016 in "Production of whey protein hydrolysates with angiotensin-converting enzyme-inhibitory activity using three new sources of vegetable proteases" describes a hydrolysate with antihypertensive activity obtained from three vegetable protease extracts (melon, citrus blossoms and from the plant *Solanum elaeagni-*

*folium.*

Although there are some studies where bromelain has been used to produce bioactive peptides from whey, there is no process that demonstrates use of bromelain and ficin that has the same steps as the process herein described. Additionally, and as it is evident throughout the description of this invention, **the use of bromelain and ficin, produce a synergistic effect in terms of production of antihypertensive activity that is greater than the technical effects of the enzyme hydrolysates that were evaluated separately.** A person skilled in this art will understand that this technical effect is unexpected and surprising, and no prior art document by itself or in combination anticipates the application herein described.

**BRIED DESCRIPTION OF THE INVENTION**

This invention describes a new process for producing bioactive antihypertensive-activity peptides comprising use of vegetable proteases, such as bromelain and ficin. The production process steps broadly correspond to the following steps:

a) Preparation of a milk serum solution,
b) pH adjustment (with NaOH 1N or with HCl 1N),
c) Preparation of the enzyme solution,
d) Heat the solution of lactic serum up to the wished temperature,
e) Addition of the required amount of enzyme solution and incubate,
f) Stop the enzyme reaction with a heat shock,
g) Centrifuge the sample and eliminate the precipitate,
h) Ultrafilter the sample, recovering the fraction under 5 kDa, and
i) Lyophilize the permeate.

This invention further relates to food compositions comprising said peptides and it also relates to uses of these compositions aimed to prevent development of cardiovascular and cerebrovascular diseases.

**DESCRIPTION OF THE FIGURES**

**Figure 1.** A calibration curve was made by using different concentrations of Captopril (x-axis) and the percentage of ACE inhibition was evaluated.

**Figure 2.** Peptide profile of the fraction under 5 kDa, obtained from hydrolysis with bromelain and heat.

**Figure 3.** Peptide profile of the fraction under 5 kDa, obtained from hydrolysis with bromelain at ambient temperature.

**Figure 4.** Peptide profile of the fraction under 5 kDa, obtained from a hydrolysis with ficin and heat.

**Figure 5.** Peptide profile of the fraction under 5 kDa, obtained from hydrolysis with ficin at ambient temperature.

**Figure 6.** Peptide profile of the fraction under 5 kDa, obtained from a hydrolysis with a blend of bromelain and ficin and heat.

**Figure 7.** Peptide profile of the fraction under 5 kDa, obtained from a hydrolysis with a blend of bromelain and ficin at ambient temperature.

**DETAILED DESCRIPTION OF THE INVENTION**

The process of producing a composition comprising bioactive antihypertensive-activity peptides from whey consists of the following steps: (a) preparing a whey solution; (b) adjusting the pH according to the reaction target; (c) preparing the enzyme solution; (d) heat up the whey solution to the desired temperature; (e) adding the required amount of enzyme solution and incubating at the reaction temperature; (f) stop the enzyme reaction with a heat shock; (g) centrifuge the sample and remove residues; (h) ultrafiltrate the sample, recovering the fraction below 5 kDa; and (i) lyophilize the permeate.

In a preferred embodiment, whey can be concentrated in order to obtain a whey-protein concentrate, such as WPC80. The term WPC80 refers to a whey-protein concentrate (80%), which results from removing non-protein fractions from whey. In order to obtain WPC, the sweet whey must go through several processing steps. The first step is clarification, which consists of removing the residual curd and fat that is usually lost during cheese making. Clarified whey is then subjected to HTST pasteurization, in order to eliminate potential pathogens and inactivate bacteria used for cheese making. Whey proteins are then concentrated, which can be done for instance by vacuum evaporation, membrane technology, such as ultrafiltration, nanofiltration, and reverse osmosis. WPC can be marketed as a liquid product or a dry powder after spray drying. WPC is widely used as an ingredient for animal feeding and for human food.

A person skilled in this art will understand that the pH setting stage can be performed with any strong acid and strong base, if the objective is to decrease or increase the pH of the reaction. In a preferred embodiment of the invention the pH values attained in this stage of the process may be between 3 and 10, preferably between 4 and 9.

The enzyme preparation stage refers to the preparation of the working solution (concentrated enzyme) to be added to the reaction. At this stage it is important to determine the enzyme units to be added to the reaction considering the amount of protein to be hydrolyzed. A person skilled in this art will understand that the enzyme activity units (U) correspond to the catalytic activity responsible for the transformation of one pmol of substrate per minute, under optimal enzyme conditions. The catalytic activity depends on each enzyme. This must be determined experimentally for each enzyme to be worked with or the enzyme supplier shall indicate such information in the protocol of use and/or in the specific datasheet. Consequently, a person skilled in this art will understand that it is not necessary to describe each of the working volumes of the reaction solutions. It is only necessary to maintain the U/mg whey protein ratio as the reaction volumes are changed.

In a preferred embodiment the enzyme activity units used of bromelain and ficin were between 0.200 U/mg to 0.400 U/mg and between 0.150/mg and 0.310 U/mg, respectively. In a more specific preferred embodiment, the values of bromelain and ficin -when used mixed- were 0.397 U/mg and 0.309 U/mg, respectively, when these were tested at 50°C.

The equilibrium step of the whey solution or WPC80, at the enzyme-reaction temperature must be performed so that the temperature of the substrate solution has reached the reaction temperature prior to the addition of the enzyme. Depending on the enzyme-reaction temperature, the equilibrium times may be different. In a preferred embodiment of the invention the equilibrium times may vary between 10 and 60 minutes.

The step of adding a solution comprising a quantity of enzyme activity units adequate for the enzymes to act well at a given temperature represents the start of the enzymatic reaction. As described herein, it is not necessary to specify the volumes used, but it is necessary to understand that the amount of enzyme units per mg of protein should be optimal, according to the supplier's instructions.

A person skilled in this art will also notice that enzymes have different performances at different temperatures. For some enzymes there is a wide temperature range in which they can hydrolyze their respective substrate, but there is a narrow temperature range in which they exert their optimum enzymatic activity. In a preferred embodiment of the invention the enzymatic reaction temperature is between 15°C and 60°C, preferably between 18°C and 55°.

In another preferred embodiment the reaction temperature is 20°C or 50°C.

Those skilled in this art will also appreciate that reaction times are different, depending on the temperature at which the reaction is carried out. That is why reaction times of this invention may vary between 3 to 70 minutes, preferably between 5 to 60 minutes.

In a more specific preferred embodiment, the reactions conducted at 20°C have a duration of about -at least- 60 minutes.

In another preferred embodiment the enzymatic reactions carried out at 50°C have a duration of about -at least- 5 minutes.

Proteins and -of course- enzymes are inactivated at high temperatures, thus losing their catalytic activity. Therefore, it is a common procedure for stopping enzymatic reactions to heat the solutions at a temperature equal to or higher than 90°C, for a time of -at least- 5 minutes. In a specific preferred embodiment, the incubation temperature is 90°C and the incubation is -at least- 5 minutes.

Although the standard protocol for stopping enzymatic reactions a person skilled in this art will understand that there are other ways to stop enzymatic reactions, such as exposing them to extreme pH ranges using strong acids, such as hydrochloric acid, or exposing them to strong bases such as sodium hydroxide. Therefore, it should not be taken as limiting to heat arrest of the enzymatic reaction, for the process described herein.

Once the reaction is finished, the centrifugation stage of the sample corresponds to a physical separation step where the particulate matter, and residues present in the whey or WPC80 and non-hydrolyzed proteins are eliminated from the subsequent stages. The supernatant containing bioactive peptides obtained is stored in a biochemical or molecular biology grade container suitable to contain said product. Although high speed centrifugation is a fast process that allows removing residues within the reaction, in order not to pass them over to subsequent steps, passive precipitation of the sample could also be performed in order to remove unwanted material.

The ultrafiltration stage uses membrane technologies with a cut-off size of 5 kDa, as an additional purification step. By means of this technology, peptides smaller than 5 kDa will pass through the membrane pores, thus forming the samples permeate, while peptides or polypeptides are retained in the upper portion of this membrane and do not pass through the pores.

Time allocated for ultrafiltration depends on the concentration of the protein sample being purified and will also depend on the volume of the sample to be passed through this ultrafiltration system.

The lyophilization step is necessary to remove water from the peptide solution. This step is essential for transporting bioactive peptides obtained at ambient temperature from the production facilities to storage facilities where the products will be stored and/or marketed. Protocols for lyophilization of protein extracts are well known in the state of the art.

In a still more specific embodiment, the process of producing the bioactive peptides consists of the following steps:

a) Prepare a solution of WPC80 at 4% powder (3.2% protein),
b) Adjust the pH of the solution obtained in (a) to 4 or 9,
c) Prepare the enzyme blend solution where the amount of bromelain and ficin are 0.397 U/mg and 0.309 U/mg, respectively,
d) Heat up the WPC80 solution to 20°C or 50°C,
e) Add the required amount of enzyme blend and incubate for 5 minutes at 60°C, or 60 minutes at 20°C,
f) Stop the enzyme reaction at 90°C for 5 minutes,
g) Centrifuge the sample between 1,000 to 15,000 RPM, preferably at 3,500 RPM, and store the supernatant in a new tube,
h) Ultrafiltrate the sample, recovering the fraction below 5 kDa; and
i) Lyophilization of the permeate.

In a preferred embodiment the bioactive peptides of this application can be used as functional ingredients for the production of different types of food, such as dairy, juices, cereal bars, baked goods, and specialized sports foods.

**Examples**

**Example #1: Production Protocols for bioactive peptides at 50°C and ambient temperature**

In order to assess the ability of bromelain and ficin enzymes to produce new bioactive peptides, each enzyme was tested separately and as a blend. In addition, an important characteristic that enzymes used in the production of this type of peptides must have is that they must work well at ambient temperature. For this reason, during this example the activity of each enzyme was evaluated separately or as a blend, at 50°C and 20°C.

The protocols used were as follows:

**Protocol at 50°C with bromelain**

A WPC80 solution with 3.2% protein is prepared. Its pH is adjusted, and the solution is prepared weight/weight with WPC80 and distilled water.

Example for a final weight of 1L of WPC solution

- 40g of WPC80 is weighed in a beaker, including stirring. Between 800g to 900g of distilled water are added. The WPC80 is dissolved under stirring for one hour at ambient temperature.
- The pH of the solution is measured, and it should be close to 6.1 - 6.3. It is adjusted to pH 9.0 with 1N NaOH or NaOH in beads or flakes.
- The weight is adjusted with distilled water until reaching a final weight of 1000 g. The WPC solution remains with a concentration of 3.2% protein.
- The solution is left stirring at ambient temperature for one hour, in order to solubilize all the protein. The pH is measured again before performing the hydrolysis.

**Hydrolysis:** Example for a hydrolysis for 1 liter of WPC80 solution.

- The two enzyme solutions are prepared, with a concentration of 26U/mL of enzyme for each one. When changing the enzyme batch, the enzyme activity must be verified on the container or on the supplier's analysis certificate, in order to ensure what the mass concentration of the solution is, which must always be 26U/mL.

- The container with the WPC80 solution is then placed in a thermoregulated bath, balanced at 50°C, which is the right reaction temperature for the bromelain/ficine blend.
- Once the WPC solution is balanced according to the reaction temperature, the volume of bromelain solution is first added. The enzyme protein ratio should be 0.397% (Enzyme unit/mg protein). For one liter of WPC solution, the volume to be added is 4.88 mL of bromelain solution, at 26U/mL. The container is stirred with WPC80 and is left in a water bath, at 50°C. The stopwatch is started. After 5 minutes, the volume of ficin solution is added. The enzyme:protein ratio should be 0.155% (Enzyme unit/mg protein). For one liter of WPC solution, the volume to be added is 1.9 mL of bromelain solution at 26U/mL. The container is stirred with the WPC80 solution and left in the water bath, at 50°C.
- After 30 seconds of ficine reaction, the container is removed and placed in another thermoregulated bath, balanced at 90 °C. The container remains in this water bath -at least 5 minutes- until its contents reach the desired temperature, in order to ensure that the enzymatic reaction has stopped.
- Centrifuge for 15 minutes, at 3500 rpm, at 4 °C in order to recover the supernatant. This centrifuged fraction will be named as "hydrolyzate" in the following protocols.
- The hydrolyzate is subsequently processed with the TFF equipment to obtain the fraction under 5 kDa.

**Protocol at ambient temperature with bromelain**

A 3.2% protein WPC80 solution is prepared and its pH is adjusted. The solution is prepared by weight/weight with WPC80 and distilled water. Example for a final weight of 1L of WPC solution:

- Weigh 40 g of WPC80 in a beaker with stirring magnet. Add 800g to 900g of distilled water and dissolve the WPC80 under stirring for one hour at ambient temperature.
- The pH of the solution is measured. It should be close to 6.1- 6.3 and adjusted to pH 9.0 with 1N NaOH or NaOH beads or flakes.
- The weight is adjusted with distilled water until a final weight of 1000 g is reached. The WPC solution is left with a concentration of 3.2% protein.
- The solution is left stirring -at ambient temperature- for one hour in order to solubilize all the protein. The pH is measured again before hydrolysis.

**Hydrolysis:** Example for a hydrolysis of 1 liter of WPC80 solution.

- The two enzyme solutions are prepared with a concentration of 26U/mL of enzyme for each one. When changing the enzyme batch, the enzyme activity must be verified on the container or on the supplier's analysis certificate in order to ensure the mass concentration of the solution, which must always be 26U/mL.
- Place the container with the WPC80 solution on the counter or in a thermoregulated bath, balanced at 20°C, which is the reaction temperature for the bromelain/ficine blend. The air conditioning and a thermometer may be used to measure the temperature of the laboratory. The temperature of the WPC80 solution should always be measured before starting any reaction.
- Once the WPC solution is balanced, according to the reaction temperature, the corresponding volumes of bromelain and ficin solution are added, at the same time. The enzyme protein ratio for bromelain should be 0.397% (Enzyme Unit/mg Protein). For one liter of WPC solution, the volume to add is 4.88 mL of bromelain solution at 26 U/m. The enzyme protein ratio for ficin should be 0.155% (Enzyme Unit/mg Protein). For one liter of WPC solution, the volume to add is 1.9 mL of bromelain solution at 26 U/mL. The container with WPC80 is stirred and left in the thermoregulated bath, at 20°C.
- After 40 minutes of reaction, the container is removed and placed in another thermoregulated bath, balanced at 90 °C. The container remains in a water bath - at least 5 minutes- until its content reaches the desired temperature, aimed to ensure that enzymatic reaction has stopped.
- The solution is centrifuged for 15 minutes, at 3500 rpm, at 4 °C and the supernatant is recovered. This centrifuged fraction will be named as "hydrolyzate" in the following protocols.
- The hydrolyzate is subsequently processed with the TFF equipment in order to obtain the fraction under 5 kDa.

**Protocol at 50°C with ficin**

A WPC80 solution with 3.2% protein is prepared. Its pH is adjusted, and the solution is prepared weight/weight with WPC80 and distilled water.

Example for a final weight of 1L of WPC solution.

- 40g of WPC80 is weighed in a beaker with a stirring magnet. Between 800g and 900g of distilled water are added and the WPC80 is dissolved under stirring for one hour at ambient temperature.
- The pH of the solution is measured. It should be close to 6.1-6.3 and adjusted to pH 4.0 with 1N HCl.
- The weight is adjusted with distilled water until reaching a final weight of 1000 g. The WPC solution remains with a concentration of 3.2% protein.
- The solution is left stirring at ambient temperature for one hour, in order to solubilize all the protein. The pH is measured again before performing the hydrolysis.

**Hydrolysis:** Example for a hydrolysis of 1 liter of WPC80 solution.

- The enzyme solution is prepared with a concentration of 26 U/mL of enzyme. When changing the enzyme batch, the enzyme activity must be verified on the container or on the supplier's analysis certificate in order to ensure the mass concentration of the solution, which must always be 26 U/mL.
- The container with the WPC80 solution is then placed in a thermoregulated bath, balanced at 50°C, which is the reaction temperature for ficine.
- Once the WPC solution is balanced, according to the reaction temperature, the corresponding volume of enzyme solution is added. The enzyme-protein ratio must be 0.3095% (Enzyme Unit/mg protein). For one liter of WPC solution, the volume to add is 3.8 mL of bromelain solution at 26 U/mL.
- The container is stirred with the WPC80 solution and left in the thermoregulated bath, at 50°C. The stopwatch is started. After 5 minutes, the container is removed and placed in another thermoregulated bath, balanced at 90 °C. The container remains in this water bath -at least 5 minutes- until its content reaches the desired temperature, to ensure that the enzymatic reaction has stopped.
- Centrifuge for 15 minutes at 3,500 rpm, at 4 °C and the supernatant is recovered. This centrifuged fraction will be named as "hydrolyzate" in the following protocols.
- The hydrolyzate is subsequently processed with the TFF equipment to obtain the fraction under 5 kDa.

**Protocol at ambient temperature with ficin**

A WPC80 solution with 3.2% protein is prepared. Its pH is adjusted, and the solution is prepared weight/weight with WPC80 and distilled water. Example for a final weight of 1L of WPC solution:

- 40g of WPC80 is weighed in a beaker with a stirring magnet. Between 800g and 900g of distilled water are added and the WPC80 is dissolved under stirring for one hour at ambient temperature.
- The pH of the solution is measured. It should be close to 6.1-6.3 and adjusted to pH 4.0 with 1N HCl.
- The weight is adjusted with distilled water until reaching a final weight of 1000 g. The WPC solution remains with a concentration of 3.2% protein.
- The solution is left stirring at ambient temperature for one hour, in order to solubilize all the protein. The pH is measured again before performing the hydrolysis.

**Hydrolysis:** Example for a hydrolysis of 1 liter of WPC80 solution.

- The enzyme solution is prepared, with a concentration of 26U/mL of enzyme. When changing the enzyme batch, the enzyme activity must be verified on the container or on the supplier's analysis certificate in order to ensure the mass concentration of the solution, which must always be 26U/mL.
- Place the container with the WPC80 solution on the counter or in a thermoregulated bath, balanced at 20°C, which is the reaction temperature for the ficine. The air conditioning and a thermometer may be used to measure the temperature of the laboratory. The temperature of the WPC80 solution should always be measured before starting any reaction.

- Once the WPC solution is balanced, according to the reaction temperature, the corresponding volume of enzyme solution is added. The enzyme-protein ratio must be 0.155% (Enzyme Unit/mg protein). For one liter of WPC solution, the volume to add is 1.9 mL of bromelain solution at 26 U/mL.
- The container is stirred with the WPC80 solution and left in the thermoregulated bath, at 50°C. The stopwatch is started. After 5 minutes, the container is removed and placed in another thermoregulated bath, balanced at 90 °C. The container remains in this water bath -at least 5 minutes- until its content reaches the desired temperature, to ensure that the enzymatic reaction has stopped.
- Centrifuge for 15 minutes at 3,500 rpm, at 4 °C and the supernatant is recovered. This centrifuged fraction will be named as "hydrolyzate" in the following protocols.
- The hydrolyzate is subsequently processed with the TFF equipment to obtain the fraction under 5 kDa

**Protocol with heat blend of enzymes**

A WPC80 solution with 3.2% protein is prepared. Its pH is adjusted, and the solution is prepared weight/weight with WPC80 and distilled water, for a final weight of 1L of WPC solution:

- 40g of WPC80 is weighed in a beaker with a magnetic stirrer. Between 800g and 900g of distilled water are added and the WPC80 is dissolved under stirring for one hour at ambient temperature.
- The pH of the solution is measured. It should be close to 6.1-6.3 and adjusted to pH 9.0 with 1N NaOH or NaOH in beads or flakes.
- The weight is adjusted with distilled water until reaching a final weight of 1000 g. The WPC solution remains with a concentration of 3.2% protein.
- The solution is left stirring at ambient temperature for one hour, in order to solubilize all the protein. The pH is measured again before performing the hydrolysis.

**Hydrolysis:** for a hydrolysis of 1 liter of WPC80 solution.

- The two enzyme solutions are prepared, with a concentration of 26 U/mL of enzyme for each one. When changing the enzyme batch, the enzyme activity must be verified on the container or on the supplier's analysis certificate in order to ensure the mass concentration of the solution, which must always be 26 U/mL.
- The container with the WPC80 solution is then placed in a thermoregulated bath, balanced at 50°C, which is the reaction temperature for the bromelain/ficine blend.
- Once the WPC solution is balanced, according to the reaction temperature, the corresponding volume of bromelain solution is first added. The enzyme protein ratio should be 0.397% (Enzyme unit/mg protein). For one liter of WPC solution, the volume to add is 4.88 mL of bromelain solution at 26 U/mL. The container is stirred with the WPC80 solution and left in the water bath at 50°C. The stopwatch is started. After 5 minutes, the corresponding volume of ficin solution is added. The enzyme protein ratio should be 0.155% (Enzyme unit/mg protein). For one liter of WPC solution, the volume to add is 1.9 mL of bromelain solution at 26 U/mL. The container is stirred with the WPC80 solution and left in the water bath at 50°C.
- After 30 seconds of ficine reaction, the container is removed and placed in another thermoregulated bath, balanced at 90 °C. The container remains in this thermoregulated bath -at least 5 minutes- until its content reaches the desired temperature, to ensure that the enzymatic reaction has stopped.
- Centrifuge for 15 minutes at 3,500 rpm, at 4 °C and the supernatant is recovered. This centrifuged fraction will be named as "hydrolyzate" in the following protocols.
- The hydrolyzate is subsequently processed with the TFF equipment to obtain the fraction under 5 kDa.

**Protocol at ambient temperature with blend of enzymes**

A WPC80 solution with 3.2% protein is prepared. Its pH is adjusted, and the solution is prepared weight/weight with WPC80 and distilled water, for a final weight of 1L of WPC solution:

- 40g de WPC80 in a beaker with fish is weighed. Add 800g to 900g of distilled water and dissolve WPC80 under stirring for one hour at ambient temperature.

- Measure the pH of the solution, which should be close to 6.1-6.3, and adjust to pH 9.0 with 1N NaOH or NaOH beads or flakes.
- Weight is adjusted with distilled water until a final weight of 1,000 g is obtained. The WPC solution is left with a concentration of 3.2% protein.
- The solution is left stirring at ambient temperature for one hour to solubilize all the protein. The pH is measured again before hydrolysis.

**Hydrolysis:** for a hydrolysis of 1 liter of WPC80 solution.

- The two enzyme solutions are prepared, with a concentration of 26 U/mL of enzyme for each one. When changing the enzyme batch, the enzyme activity must be verified on the container or on the supplier's analysis certificate, in order to ensure the mass concentration of the solution, which must always be 26 U/mL.
- Place the container with the WPC80 solution on the counter or in a thermoregulated bath, balanced at 20°C, which is the reaction temperature for the bromelain/ficine blend. The air conditioning and a thermometer may be used to measure the temperature of the laboratory. The temperature of the WPC80 solution should always be measured before starting any reaction.
- Once the WPC solution is balanced, according to the reaction temperature, the corresponding volumes of bromelain and ficin solution are added at the same time. The enzyme protein ratio for bromelain should be 0.397% (Enzyme Unit/mg Protein). For one liter of WPC solution, the volume to add is 4.88 mL of bromelain solution at 26 U/m. The enzyme protein ratio for ficin should be 0.155% (Enzyme Unit/mg Protein). For one liter of WPC solution, the volume to add is 1.9 mL of bromelain solution at 26 U/mL. The container is stirred with the WPC80 solution and left in the water bath at 50°C.
- After 40 minutes of reaction, the container is removed and placed in another thermoregulated bath, balanced at 90 °C. The container remains in this thermoregulated bath -at least 5 minutes- until its content reaches the desired temperature, to ensure that the enzymatic reaction has stopped.
- Centrifuge for 15 minutes at 3,500 rpm, at 4 °C and the supernatant is recovered. This centrifuged fraction will be named as "hydrolyzate" in the following protocols.
- The hydrolyzate is subsequently processed with the TFF equipment to obtain the fraction under 5 kDa.

Then, the samples were lyophilized to be stored at ambient temperature. The lyophilization protocol consisted of the following steps: 1) Freeze the samples, at 34°C for -at least- 8 hours and 2) lyophilize the samples using the parameters described in Table 1.

Table 1. Lyophilization parameters of peptides

| Hour | Chamber vacuum (kPa)mtorr | Temperature Condensator (°C) | Temperature Trays (°C) | Set-Point Trays (°C) | Ambient Temperature (°C) |
|---|---|---|---|---|---|
| 23:40 | --------- | -49,2 | -0,7 | --------- | 17 |
| 00:16 | 30.66 | -37.8 | -33 | -20 | 17 |
| 00:26 | 18.40 | -42.8 | -26 | -20 | 18 |
| 07:30 | 20.53 | -48.2 | -16 | -10 | 15 |
| 08:20 | 22.4 | -47.3 | -9.6 | 0 | 17 |
| 09:10 | 23.06 | -45.3 | -3.8 | 0 | 19 |
| 10:09 | 24.26 | -45.3 | -3.6 | 0 | 20 |

(continued)

| Hour | Chamber vacuum (kPa)mtorr | Temperature Condensator (°C) | Temperature Trays (°C) | Set-Point Trays (°C) | Ambient Temperature (°C) |
|---|---|---|---|---|---|
| 11:31 | 25.33 | -44.1 | -0.8 | 0 | 23 |
| 12:29 | 25.46 | -42.9 | -2.9 | 0 | 24 |
| 14:03 | 25.73 | -40.0 | -2.2 | 0 | 24 |
| 15:04 | 26.00 | -39.9 | -1.3 | 0 | 25 |
| 17:59 | 26.66 | -39.8 | +0.3 | 0 | 25 |
| 19:00 | 26.80 | -40.0 | +2.3 | +15 | 24 |
| 22:30 | 26.40 | -42.4 | +13 | +15 | 21 |
| 23:15 | 24.93 | -43.8 | +18 | +25 | 19 |
| 24:15 | 22.00 | -46.7 | +25 | +25 | 14 |

**Example # 2: Determination of antihypertensive activity and equivalence curve with Captopril (gold standard)**

The method for measuring spectrophotometry antihypertensive activity is based on the release of hippuric acid from hippuryl-histidyl-leucine (HHL), a reaction catalyzed by angiotensin-converting enzyme (ACE). The presence of bioactive angiotensin inhibitory peptides, such as those expected to be found in dairy products, are expected to inhibit this enzymatic pathway (Lu et al., 2016). Inhibiting ACE activity inhibits the release of hippuric acid, an analyte that is detected by absorbance at 228nm.

**Reactives**

-     An ideally fresh borate buffer is prepared. However, 50 mL of buffer can be prepared and 200 $\mu$L of buffer is distributed in Eppendorf tubes and kept frozen until the test day. A buffer at 0.1M sodium borate, 0.3M NaCl and 5 mM Hippuryl-Histidyl-Leucine is prepared. In order to prepare 50mL of buffer, 1.91 g of sodium borate, 0.877 g of NaCl and 0.107 g of HHL are weighed. This blend is dissolved in 50 mL of milliQ water, using a volumetric flask. Heat can be applied for a few minutes if it is difficult to solubilize the buffer. Once dissolved, the pH is adjusted to 8.3 with 1N HCl.

    -     A solution of ACE 0.25 Units/mL is prepared: Refer to the ACE analysis certificates to make the dilution. The diluted ACE solution is separated into 0.5 mL aliquots and frozen until used.

**Protocol**

• The samples are placed in a water bath -at 37°C- for one hour to thaw them.
• Eppendorf tubes containing 200 $\mu$L of borate buffer are removed from the freezer. They are noted as follows: a single tube A, a single tube B and a tube C and a tube D for each sample. Tubes A and B are used as a reference for the entire analysis. Tubes C and D are used to measure the antihypertensive capacity of each sample. The following table details the contents of each tube. When no sample or ACE solution needs to be added, the same volume of milliQ water is added to the corresponding tubes.

| Tube | A | B | C | D |
|------|------|------|------|------|
| Sample | NO | NO | SI | SI |
| ACE | YES | NO | YES | NO |

• 50 μL of sample is added to each tube C and D, while 50 μL of milliQ water is added to tube A and tube B. It is passed through Vortex.
• The tubes remain in a water bath -at 37°C- for 5 minutes in order to balance the reaction temperature.
• 20 μL of milliQ water are added to tubes D and tube B: The tubes are closed and passed through Vortex.
• 20 μL of ACE solution is added to tubes C and tube A. They are closed and passed through Vortex. No drops must remain on the walls of the tube. All of the liquid must be at the bottom of the tube to ensure a good reaction.
• It remains in the thermoregulated bath at 37°C, for 30 minutes.
• To stop the reaction, 250 μL of 1 N HCl are added. The tubes are passed through Vortex.
• Under the hood, 1.4 mL of ethyl acetate is added. The tubes are stirred. As ethyl acetate is an organic solvent, it allows hippuric acid to be separated from water. This acid is released during the reaction. It will then be measured to determine the antihypertensive capacity of the samples.
• The tubes are centrifuged at 729 g x 10 minutes (2,500 rpm).
• Under a hood, 1 mL of the supernatant is removed and transferred to new Eppendorf tubes scored in the same way.
• The supernatant is allowed to evaporate completely by placing the open Eppendorf tubes in a pot of boiling water. In general, the complete evaporation process takes 15 - 20 minutes. The entire process must be carried out under a hood, as ethyl vapors are released.
• Once the tubes have been completely evaporated, they are removed from the pot and 1 mL of milliQ water is added. They are passed through Vortex.
• The content of the tubes is transferred to UV cuvettes and the absorbance is measured at 228 nm.

**Calculations**

Values of A, B, C and D are absorbance values determined for each tube. As there is only one tube A and one tube B, the same values are used for all samples. In order to calculate the $IC_{50}$, the value of the peptide/amino acid concentration of the fraction below 3 kDa is used, as well as the percentage inhibition calculated for each sample.

$$\text{Inhibition value (\%)} = \left[ 1 - \left( \frac{C - D}{A - B} \right) \right] \times 100$$

$$IC_{50} = \frac{Sample\ Concentration\ \left( \frac{mgLeucine}{L\ sample} \right) x\ 50}{\%\ ACE\ Inhibition\ of\ sample}$$

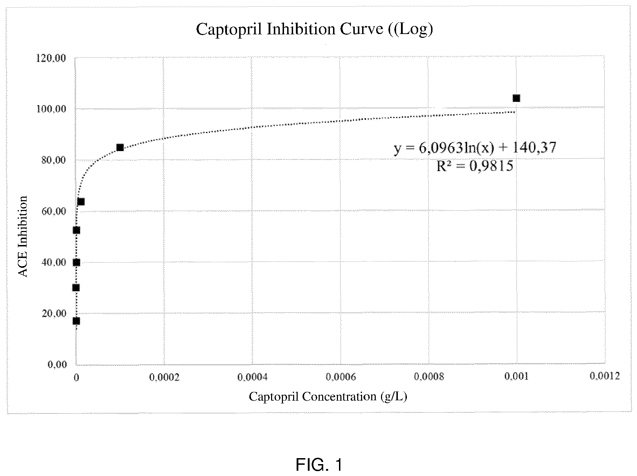

Captopril is a drug with an inhibitory effect on ACE. It is used in human medicine as a treatment for hypertensive people. In order to have a reference point aimed to compare the antihypertensive effects of the different extracts, a calibration curve of Captopril was made. The objective was to correlate a percentage of ACE inhibition with a concentration of Captopril. Results were obtained proving a high inhibitory power of Captopril on ACE, with a logarithmic curve with an asymptote at 100% inhibition. The equation of the calibration curve was obtained, which is used to obtain the equivalents of Captopril for the extracts tested in this project (Figure 1).

**Example # 3: Characterization of peptide extracts obtained and comparison with Captopril**

The peptide fractions obtained in Example #1 were evaluated for their antihypertensive capacity, according to the protocol described in the previous example. The antihypertensive capacity of the samples obtained with different treatments was evaluated: (1) bromelain with heat, (2) bromelain at ambient temperature, (3) ficin with heat, (4) ficin at ambient temperature, (5) enzymes blend with heat, and (6) enzyme blend at ambient temperature. The results obtained are shown in the following tables 2-7:

Table 2: Bromelain with heat

|  | BRO - Heat |
| --- | --- |
| Hydrolysis Conditions | |
| Raw material | WPC80 solution at 3.2% of protein |
| Temperature (°C) | 50 |
| pH | 9.0 |
| Reaction time (min) | 5 |
| Enzyme % (U enzyme/mg protein) | 0.397 |
| Filtration time (to obtain one liter of fraction under 5 kDa) | 112 minutes |
| Features of the fraction under 5 kDa | |
| % ACE inhibition | 86.7 |
| Peptide concentration (mgLeucine/L) | 153.96 |
| Liquid Captopril Equivalent (for 1 mg of Captopril) | 6.7 mL |
| Lyophilized Captopril Equivalent (for 1 mg of Captopril) | 12.9 mg |
| Dry Matter | 0.19 |
| Appearance lyophilized powder | Orange/yellow, sticky |

Table 3. Bromelain at ambient temperature

|  | BRO - Ambient Temperature |
| --- | --- |
| Hydrolysis Conditions | |
| Raw material | WPC80 solution at 3.2% of protein |
| Temperature (°C) | 20 |
| pH | 9.0 |
| Reaction time (min) | 60 |
| Enzyme % (U enzyme/mg protein) | 0.397 |
| Filtration time (to obtain one liter of fraction under 5 kDa) | 109 minutes |
| Features of the fraction under 5 kDa | |
| % ACE inhibition | 74.9 |
| Peptide concentration (mgLeucine/L) | 244.58 |
| Liquid Captopril Equivalent (for 1 mg of Captopril) | 46.1 mL |
| Lyophilized Captopril Equivalent (for 1 mg of Captopril) | 87.8 mg |
| Dry Matter | 0.19 |
| Appearance of lyophilized powder | White/Yellow |

Table 4. Ficin with Heat

|  | FIC - Heat |
| --- | --- |
| Hydrolysis Conditions | |
| Raw material | WPC80 solution at 3.2% of protein |
| Temperature (°C) | 50 |

(continued)

| | FIC - Heat |
|---|---|
| **Hydrolysis Conditions** | |
| pH | 4.0 |
| Reaction time (min) | 4 |
| Enzyme % (U enzyme/mg protein) | 0.3095 |
| Filtration time (to obtain one liter of fraction under 5 kDa) | 100 minutes |
| **Features of the Fraction Under 5 kDa** | |
| % ACE inhibition | 91.0 |
| Peptide concentration (mgLeucine/L) | 352.12 |
| Liquid Captopril Equivalent (for 1 mg of Captopril) | 3.3 mL |
| Lyophilized Captopril Equivalent (for 1 mg of Captopril) | 18.5 mg |
| Dry Matter | 0.56 |
| Appearance of lyophilized powder | White/Yellow |

Table 5. Ficin at ambient temperature

| | FIC - Ambient Temperature |
|---|---|
| **Hydrolysis Conditions** | |
| Raw material | WPC80 solution at 3.2% of protein |
| Temperature (°C) | 20 |
| pH | 4.0 |
| Reaction time (min) | 60 |
| Enzyme % (U enzyme/mg of protein) | 0.155 |
| Filtration time (to obtain one liter of fraction under 5 kDa) | |
| **Features of the Fraction Under 5 kDa** | |
| % ACE inhibition | 92.4 |
| Peptide concentration (mg Leucine/L) | 333.6 |
| Liquid Captopril Equivalent (for 1 mg of Captopril) | 2.6 mL |
| Lyophilized Captopril Equivalent (for 1 mg of Captopril) | 14.9 mg |
| Dry Matter | 0.57 |
| Appearance lyophilized powder | White/Yellow |

Table 6: Blend of enzymes with Heat

| | MEZ - Heat |
|---|---|
| **Hydrolysis Conditions** | |
| Raw material | WPC80 solution at 3.2% of protein |
| Temperature (°C) | 50 |
| pH | 9.0 |
| Reaction time (min) | 5 minutes BRO + 30 minutes FIC |

(continued)

| | MEZ - Heat |
|---|---|
| Hydrolysis Conditions | |
| Raw material | WPC80 solution at 3.2% of protein |
| Enzyme % (U enzyme/mg of protein) | 0.397 BRO + 0.155 FIC |
| Filtration time (to obtain one liter of fraction under 5 kDa) | 138 minutes |
| Features of the Fraction Under 5 kDa | |
| % ACE inhibition | 93.5 |
| Peptide concentration (mgLeucine/L) | 360.58 |
| Liquid Captopril Equivalent (for 1 mg of Captopril) | 2.2 mL |
| Lyophilized Captopril Equivalent (for 1 mg of Captopril) | 12.0 mg |
| Dry Matter | 0.55 |
| Appearance lyophilized powder | White/Yellow - "Cotton candy" appearance |

Table 7. Blend of enzymes at Ambient Temperature

| | MEZ - Ambient Temperature |
|---|---|
| Hydrolysis Conditions | |
| Raw material | WPC80 solution at 3.2% of protein |
| Temperature (°C) | 20 |
| pH | 9.0 |
| Reaction time (min) | 40 minutes |
| Enzyme % (U enzyme/mg of protein) | At the same time 0.397 BRO + 0.155 FIC |
| Filtration time (to obtain one liter of fraction under 5 kDa) | 150 minutes |
| Features of the Fraction Under 5 kDa | |
| % ACE inhibition | 95.7 |
| Peptide concentration (mgLeucine/L) | 353.33 |
| Liquid Captopril Equivalent (for 1 mg of Captopril) | 1.5 mL |
| Lyophilized Captopril Equivalent (for 1 mg of Captopril) | 11.0 mg |
| Dry Matter | 0.72 |
| Appearance lyophilized powder | White/Yellow - "Cotton candy" appearance |

The results presented in this table demonstrate that when enzymes are mixed together they generate peptide extracts with higher antihypertensive activity as compared to using each enzyme separately. These results demonstrate the synergy of both enzymes, being these results neither expected nor derivable from the state of the art. In addition, the antihypertensive activity of the peptide extract obtained by a production process that uses a mixture of enzymes and carried out at ambient temperature is superior to that of the same production process carried out at 50°C. It should be noted that a production process carried out at room temperature generates economic advantages by not having to heat the solution to carry out the enzymatic reaction.

**Example # 4: Peptide profiling of the samples** - **HPLC**

Fractions lower than 5 kDa. obtained with the protocols described in the previous examples were analyzed. Samples were prepared by mixing 400uL of the fraction below 5 kDa and adjusting with 1.6 mL with milliQ water with 0.1% trifluoroacetic acid (TFA) to reach a total volume of 2 mL. Samples were filtered with $0.45\mu$m pyrindole filter directly into vials for HPLC analysis. Peptides were analyzed at a wavelength of 214 nm. The phase consists of two solvents: A, milliQ water with 0.1% TFA, and B, acetonitrile with 0.1% TFA. The elution is controlled for 60 min, with a mobile phase flow rate of 0.75mL/min, with the oven balanced at 40°C. Two blanks (milliQ water) are run before analyzing samples, in order to remove possible residues from previous samples. A blank (milliQ water) is run every third sample to clean the system. The analysis batch is terminated with two more blanks to clean the system and leave it operational for subsequent samples.

In order to analyze the chromatograms, all of them were standardized with the "Background" tool (Shimadzu Corporation, City, Country), which is used to smooth the baseline with a reference chromatogram obtained by analyzing a milliQ water blank. Ideally, a blank that had not been run as the first sample in the system.

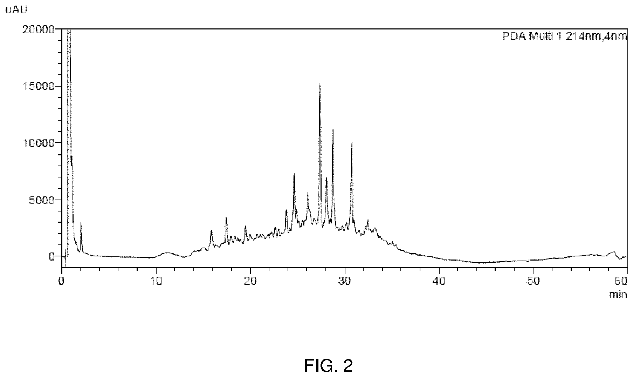

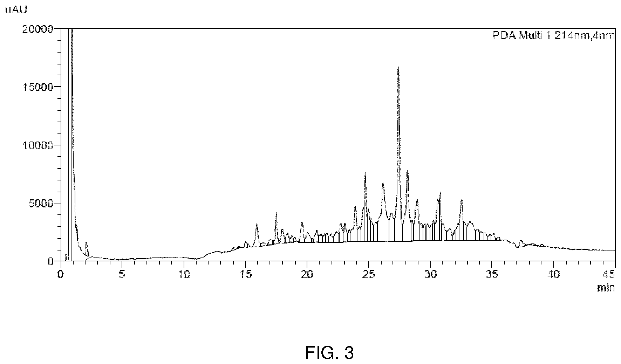

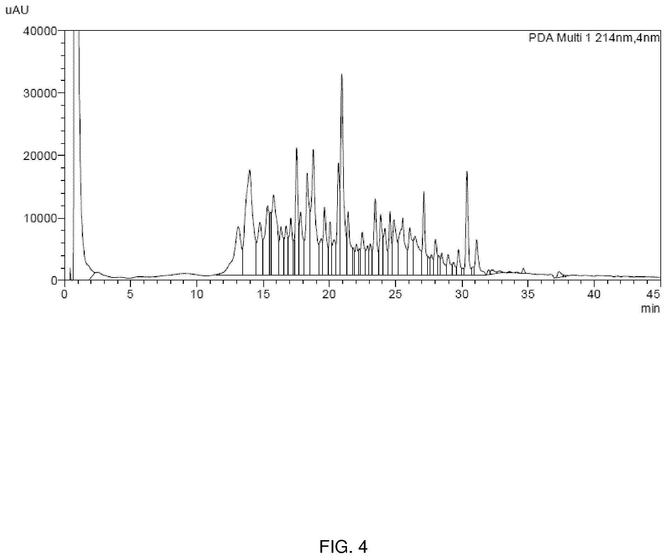

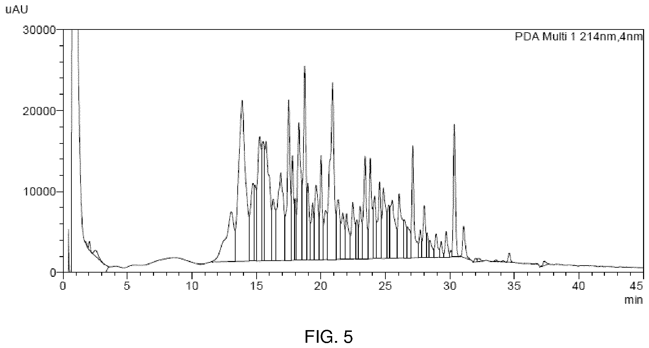

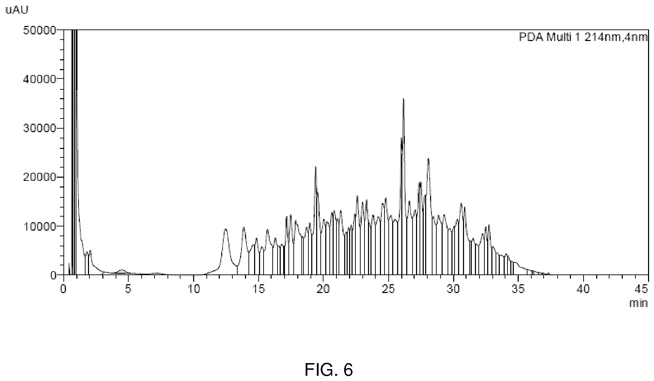

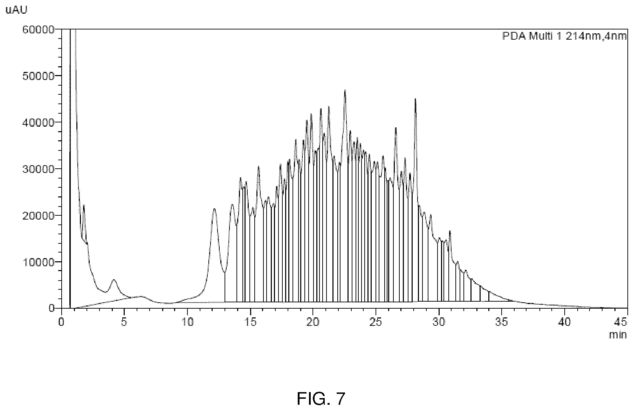

Peptide profiles were obtained for fractions below 5 kDa for each sample. The chromatograms are presented in Figures 2-7, where in each of these chromatograms the highest peaks were highlighted. These chromatograms serve as reference and quality control for all fractions obtained in the project.

As noticed from the chromatograms in Figures 2-7, the samples obtained by using the enzymes bromelain and ficin, which were assayed at room temperature, show the highest diversity of peptides (evidenced by the number of peaks in the chromatogram), and also show the highest abundance of peptides.

**This is the first report of a whey-derived peptide extract that exhibits these antihypertensive properties, as demonstrated throughout the present application, by using the enzymes bromelain and ficin, and which is made at ambient temperature.**

## INDUSTRIAL APPLICATION

The herein invention has a great application in the food industry, specifically in the field of functional ingredients and nutraceuticals which may be used as ingredients in different types of food for the purpose of preventing development of cardiovascular problems.

## Claims

1. A process, for the production of bioactive peptides with antihypertensive activity, wherein it includes the following steps:

   a) prepare the whey solution;
   b) adjust the pH of the obtained solution;
   c) prepare a solution containing a blend of bromelain and ficin enzymes;
   d) bring the whey solution to a temperature of -at least- 15°C;
   e) add the required amount of the enzyme blend obtained in c) and incubate for 5 min, at 50°c, or 60 min at 20°C;
   f) stop the enzymatic reaction with a heat shock, at a temperature of -at least-90°C;
   g) optionally centrifuge the sample and store the supernatant in a new tube;
   h) ultrafilter the sample, recovering the fraction below 5 kDa; and
   i) lyophilizate the permeate.

2. The process according to claim 1, wherein whey solution is a whey-protein concentrate, wherein said whey-protein concentrate is selected from WPC10, WPC20, WPC30, WPC40, WPC50, WPC60, WPC70, WPC80 o WPC90.

3. The process according to claims 1 or 2, wherein the pH of the solution is adjusted to a value between -at least- 4 to 9.

4. The process according to any of the claims 1 to 3, wherein bromelain is added in an amount between 0.200 U/mg protein to 0.400 U/mg protein.

5. The process according to any of the claims 1 to 4, wherein ficin is added in an amount between 0.150/mg protein and 0.310 U/mg protein.

6. The process according to any of the claims 1 to 5, wherein bromelain is added at 0.400 U/mg protein.

7. The process according to any of the claims 1 to 6, wherein Ficin is added at 0.309 U/mg of protein.

8. The process according to any of the claims 1 to 7, wherein the stage (d) is carried out at a temperature between 15°C to 60°C.

9. The process, according to claim 8, wherein the stage (d) is carried out at a temperature between 20°C to 50°C.

10. The process according to any of the claims 1 to 9, wherein the reaction of the stage e) is carried out for at least 3 minutes.

11. The process according to any of the claims 1 to 10, wherein the reaction of the stage e) is carried out between 3 to 60 minutes.

12. The process according to any of the claims 1 to 11, wherein the heat arrest of the reaction of step (f) is performed by incubation, at a temperature of -at least- 90°C, for -at least- 3 min.

13. The process according to any of the claims 1 to 12, wherein the arrest of the heat reaction of step (f) is performed by incubation, at a temperature of -at least- 90°C between 3 to 15 minutes.

14. The process according to any of the claims 1 to 13, wherein the centrifugation stage (g) is performed at a speed between 1,000 RPM and 15,000 RPM.

15. A functional ingredient, wherein it comprises bioactive peptides obtained by the process, according to any of the claims 1 to 14, and excipients acceptable in the food industry.

16. The functional ingredient, according to claim 15, wherein it can be used to prepare different types of food, such as dairy products, juices, cereal bars, baked products, and specialized foods for athletes.

FIG. 1

FIG. 2

FIG. 3

FIG. 4

FIG. 5

FIG. 6

FIG. 7

## INTERNATIONAL SEARCH REPORT

| International application No. |
|---|
| PCT/CL2021/050064 |

**A.   CLASSIFICATION OF SUBJECT MATTER**

CIP: A23L33/18, A23J3/34, A61K38/17, C12P21/06 (2022.01)

According to International Patent Classification (IPC) or to both national classification and IPC

**B.   FIELDS SEARCHED**

Minimum documentation searched (classification system followed by classification symbols)

CIP: A23L33/18, A23J3/34, A61K38/17, C12P21/06

Documentation searched other than minimum documentation to the extent that such documents are included in the fields searched

Electronic data base consulted during the international search (name of data base and, where practicable, search terms used)

INAPI, STN, GOOGLE, PATENTSCOPE

**C.   DOCUMENTS CONSIDERED TO BE RELEVANT**

| Category* | Citation of document, with indication, where appropriate, of the relevant passages | Relevant to claim No. |
|---|---|---|
| X | TONDO, A., et al. Structure-based identification and design of angiotensin converting enzyme-inhibitory peptides from whey proteins. Journal of Agricultural and Food Chemistry, 2020, vol. 68, no 2, p. 541-548. DOI: 10.1021/acs.jafc.9b06237 **The whole document** | |
| A | IWANIAK, A., et al. Hybrid approach in the analysis of bovine milk protein hydrolysates as a source of peptides containing di-and tripeptide bitterness indicators. Polish Journal of Food and Nutrition Sciences, 2020, vol. 70, no 2. DOI: 10.31883/pjfns/113532 **The whole document** | |
| A | CHEUNG, L., et al. Effects of exopeptidase treatment on antihypertensive activity and taste attributes of enzymatic whey protein hydrolysates. Journal of Functional Foods, 2015, vol. 13, p. 262-275. DOI: 10.1016/j.jff.2014.12.036 **The whole document** | |

☒  Further documents are listed in the continuation of Box C.        ☒  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11/04/2022      11/ April/2022 | 18/04/2022     18/ April/2022 |

| Name and mailing address of the ISA/ INAPI, Av. Libertador Bernardo O'Higgins 194, Piso 17, Santiago, Chile | Authorized officer |
|---|---|
| Facsimile No. | Telephone No. |

Form PCT/ISA/210 (second sheet) (January 2015)

**INTERNATIONAL SEARCH REPORT**

| International application No. |
|---|
| PCT/CL2021/050064 |

C (Continuation).     DOCUMENTS CONSIDERED TO BE RELEVANT

| Category* | Citation of document, with indication, where appropriate, of the relevant passages | Relevant to claim No. |
|---|---|---|
| A | MEDEIROS, V., et al. Bovine milk formula based on partial hydrolysis of caseins by bromelain enzyme: Better digestibility and angiotensin-converting enzyme-inhibitory properties. International journal of food properties, 2014, vol. 17, no 4, p. 806-817. DOI: 10.1080/10942912.2012.675607<br>The whole document | |
| A | JEEWANTHI, R., et al. Improved functional characteristics of whey protein hydrolysates in food industry. Korean journal for food science of animal resources, 2015, vol. 35, no 3, p. 350. DOI: 10.5851/kosfa.2015.35.3.350 | |

Form PCT/ISA/210 (continuation of second sheet) (January 2015)

## REFERENCES CITED IN THE DESCRIPTION

**Patent documents cited in the description**

- CN 111944009 A
- JP 2020097535 A
- CN 108178785 A
- KR 20110089705 A
- JP 2002238462 A
- GB 966857 A

**Non-patent literature cited in the description**

- **PRATHAMESH BHARAT HELKAR ; AK SAHOO ; NJ PATIL.** Review: Food Industry By-Products used as a Functional Food Ingredients. *Int J Waste Resour 2016,* 2016, vol. 6, 3
- **KORHONEN ; PIHLANTO.** Food-derived Bioactive Peptides - Opportunities for Designing Future Foods. *Current Pharmaceutical Design,* 2006, vol. 9 (16), 1297-308
- **TAVARES ; MALCATA.** *Whey Proteins as Source of Bioactive Peptides Against Hypertension,* 2013
- **TORRUCO-UCO et al.** Angiotensin-I converting enzyme inhibitory and antioxidant activities of protein hydrolysates from Phaseolus lunatus and Phaseolus vulgaris sedes. *Food Science and Technology.,* 2009, vol. 42 (10), 1597-1604
- **BEN HENDA et al.** Measuring Angiotensin-I Converting Enzyme Inhibitory Activity by Micro Plate Assays: Comparison Using Marine Cryptides and Tentative Threshold Determinations with Captopril and Losartan. *J. Agric. Food Chem.,* 2013, vol. 61 (45), 10685-10690
- **MAZORRA-MANZANO, M. et al.** *Production of whey protein hydrolysates with angiotensin-converting enzyme-inhibitory activity using three new sources of vegetable proteases,* 2016